# EUROPEAN PATENT APPLICATION

(11) **EP 4 807 761 A1**
(43) Date of publication of application: **16.09.2026**
(21) Application number: 25190340.7
(22) Date of filing: 18.07.2025
(51) Int. Cl.: G16H 50/50, A61C 19/06

(54) **METHOD, APPARATUS, AND COMPUTER-READABLE RECORDING MEDIUM FOR DESIGNING MOUTH SHIELD HAVING MEDICAMENT APPLICATION AREA**

(30) Priority: 10.03.2025 KR 20250030558
(71) Applicant: Innodtech, Inc., Gwangju (KR)
(72) Inventor: JOO, Bo Hoon, 06004 Seoul (KR)
(74) Representative: Kaya, Erdem

(57) **Abstract**

A method for designing a mouth shield (100) having a medicament application area includes: an object classification and identification step (S10) of classifying and identifying objects corresponding to a tooth part (30T) and a gum part (30G) from oral scan data (1); a temporary model generation step (S20) of generating a temporary model by performing modeling on the mouth shield (100) having a shape to cover the tooth part (30T) and the gum part (30G) identified in the object classification and identification step (S10) using a design program; and a correction model generating step (S30) of generating a correction model, in which correction is performed on the temporary model in which a design for the temporary model is corrected such that a medicament application surface is secured in consideration of the application thickness of the medicament (M) to be applied to an inner area of the mouth shield, thereby generating the correction model with optimized consistency of the mouth shield.

## Description

### BACKGROUND OF THE INVENTION

### 1. Field of the Invention

The present invention relates to a method for designing a mouth shield having a medicament application area, and more specifically, to a technology of designing a mouth shield to achieve the purpose of enhancing health by a medicament, by continuously supplying the medicament that is applied to a preset area of the mouth shield in order to enhance oral health in an oral cavity.

### 2. Description of the Related Art

Currently, various types of technologies for delivering a medicament into an oral cavity are being developed and commercialized in the field of dentistry and oral treatment.

Examples of the technologies largely include an oral rinsing solution-based medicament delivery method and an application-type medicament-based medicament delivery method, in which the oral rinsing solution-based medicament delivery method is a method in which a user keeps an oral rinsing solution containing ingredients such as an antibacterial agent, fluorine, and an anti-inflammatory agent in the oral cavity for a certain time and then spits the oral rinsing solution out to remove bacteria in the oral cavity and prevent periodontal diseases. However, the oral rinsing solution-based medicament delivery method has problems in which repeated use of the oral rinsing solution is required since the medicament ingredients do not stay in the oral cavity for a long time and are rapidly removed together with saliva, the effect disappears after a predetermined time, and it is difficult to intensively deliver the medicament to a measurement site in the oral cavity.

In addition, the application-type medicament-based medicament delivery method is a method of directly applying fluorogel, antibiotic ointment, analgesic gel, or the like to a specific site with a finger or a cotton swab. However, the method has a problem in which the fluorogel, antibiotic ointment, analgesic gel, or the like is easily diluted or removed by saliva and food in the oral cavity, resulting in a limitation that the treatment effect is not significant because continuous application thereof is also required.

In order to overcome the limitations, Korean Registered Patent No. 10-2569589 (oral sealing wrap having elasticity) discloses a technology for delivering a patch-type medicament that is a product for oral care and includes a layer having a surface in contact with teeth or tissues around the teeth, and allows the medicament applied to a target site in the oral cavity to stay for a desired time.

However, in the case of the prior art, there may be a problem that the oral sealing wrap easily falls before a target time according to an oral environment (amount of saliva secretion, habit) of the user, and there is a limitation in that the medicament delivery effect is only insignificant because the medicament is not sufficiently applied to the target site due to the anatomical structure of the teeth or gums different for each user.

### SUMMARY OF THE INVENTION

Accordingly, an object of the present invention is to achieve the purpose of enhancing oral health of a user who wears a mouth shield by continuously supplying a medicament, which is prepared to enhance oral health in an oral cavity, to a target site set in the oral cavity.

To achieve the above object, according to one embodiment of the present invention, a method for designing a mouth shield having a medicament application area, which is implemented by a computing device including one or more processors and one or more memories for storing instructions executable in the processors, includes: an object classification and identification step of classifying and identifying objects corresponding to a tooth part and a gum part from oral scan data, which is obtained by three-dimensionally scanning an oral condition of a subject, using a preset object extraction algorithm; a temporary model generation step of generating a temporary model by performing modeling using a design program on the mouth shield having a shape to cover the tooth part and the gum part identified in the object classification and identification step; a correction model generation step of generating a correction model, in which correction is performed on the temporary model wherein an artificial intelligence model calculates an application thickness of a medicament to be applied to an inner area of the mouth shield based on the oral scan data and oral examination information of the subject, and a design of the temporary model is corrected such that a medicament application surface having a thickness corresponding to the calculated result is secured, thereby generating the correction model with optimized consistency of the mouth shield, so that a 3D printing-based molding process is performed based on design data of the correction model, in which the medicament application surface formed on the mouth shield includes a release film disposed in a form of tightly covering the medicament application surface, in which the release film has a structure in which an inside thereof includes a plurality of pores so that the medicament applied to the medicament application surface is delivered to the tooth part and the gum part of the subject through the plurality of pores, and the release film includes: an outer layer formed of a material including at least one of a polyurethane membrane and a fluororesin and having a structure including a plurality of pores; and a hydrophilic hydrogel-based absorbent layer configured to adjust a discharge rate of the medicament by absorbing and storing the medicament applied to the medicament application surface between the medicament application surface and the outer layer.

In this case, the method may further include an oral examination information collection step of collecting oral examination information including at least one of age, body weight, gum condition, and severity of a disease of the subject as the oral examination information of the subject, before the correction model generation step, so that an appropriate dose of a medicament required for the subject may be calculated according to the collected oral examination information, and in the correction model generation step, the design of the temporary model may be corrected by determining a thickness of the medicament application surface corresponding to the calculated dose of the medicament.

In addition, in the correction model generation step, the correction model may be generated by changing the design such that a predetermined separation space for securing the medicament application surface is formed in a direction including at least one of a lingual side and a labial side of the tooth part in the inner area of the temporary model.

In addition, the medicament application surface formed on the mouth shield may be an area including at least one of an entire inner area forming a contact surface with the tooth part and the gum part of the subject or a local area corresponding to a gingival margin part, which is a boundary between the tooth part and the gum part of the subject.

Meanwhile, according to one embodiment of the present invention, an apparatus for designing a mouth shield having a medicament application area, which is implemented by a computing device including one or more processors and one or more memories for storing instructions executable in the processors, includes: an object classification and identification unit configured to classify and identify objects corresponding to a tooth part and a gum part from oral scan data, which is obtained by three-dimensionally scanning an oral condition of a subject, using a preset object extraction algorithm; a temporary model generation unit configured to generate a temporary model by performing modeling using a design program on the mouth shield having a shape to cover the tooth part and the gum part identified by the object classification and identification unit; a correction model generation unit configured to generate a correction model, in which correction is performed on the temporary model wherein an artificial intelligence model calculates an application thickness of a medicament to be applied to an inner area of the mouth shield based on the oral scan data and oral examination information of the subject, and a design of the temporary model is corrected such that a medicament application surface having a thickness corresponding to the calculated result is secured, thereby generating the correction model with optimized consistency of the mouth shield, so that a 3D printing-based molding process is performed based on design data of the correction model, in which the medicament application surface formed on the mouth shield includes a release film disposed in a form of tightly covering the medicament application surface, in which the release film has a structure in which an inside thereof includes a plurality of pores so that the medicament applied to the medicament application surface is delivered to the tooth part and the gum part of the subject through the plurality of pores, and the release film includes: an outer layer formed of a material including at least one of a polyurethane membrane and a fluororesin and having a structure including a plurality of pores; and a hydrophilic hydrogel-based absorbent layer configured to adjust a discharge rate of the medicament by absorbing and storing the medicament applied to the medicament application surface between the medicament application surface and the outer layer.

Meanwhile, a computer-readable recording medium that stores instructions for allowing a computing device to perform the following steps, in which the steps include: an object classification and identification step of classifying and identifying objects corresponding to a tooth part and a gum part from oral scan data, which is obtained by three-dimensionally scanning an oral condition of a subject, using a preset object extraction algorithm; a temporary model generation step of generating a temporary model by performing modeling using a design program on the mouth shield having a shape to cover the tooth part and the gum part identified in the object classification and identification step; a correction model generation step of generating a correction model, in which correction is performed on the temporary model wherein an artificial intelligence model calculates an application thickness of a medicament to be applied to an inner area of the mouth shield based on the oral scan data and oral examination information of the subject, and a design of the temporary model is corrected such that a medicament application surface having a thickness corresponding to the calculated result is secured, thereby generating the correction model with optimized consistency of the mouth shield, so that a 3D printing-based molding process is performed based on design data of the correction model, in which the medicament application surface formed on the mouth shield includes a release film disposed in a form of tightly covering the medicament application surface, in which the release film has a structure in which an inside thereof includes a plurality of pores so that the medicament applied to the medicament application surface is delivered to the tooth part and the gum part of the subject through the plurality of pores, and the release film includes: an outer layer formed of a material including at least one of a polyurethane membrane and a fluororesin and having a structure including a plurality of pores; and a hydrophilic hydrogel-based absorbent layer configured to adjust a discharge rate of the medicament by absorbing and storing the medicament applied to the medicament application surface between the medicament application surface and the outer layer.

According to one embodiment of the present invention, in order to enhance oral health in the oral cavity, the medicament applied to the medicament application surface of the mouth shield is continuously delivered to the target site of the subject, so that a therapeutic effect may be maintained for a long time compared to a method for supplying a medicament like gargling, thereby rapidly achieving the purpose of enhancing oral health.

In addition, according to one embodiment of the present invention, it is possible to expect an efficient local therapeutic effect by setting the medicament application surface, which is formed on the mouth shield, as a surface corresponding to the gingival margin line of the subject.

In addition, according to one embodiment of the present invention, the present invention does not simply apply the medicament to the medicament application surface formed on the mouth shield to provide the medicament to the subject, but applies the medicament to the medicament application surface and tightly covers the medicament application surface with a release film, so that it is possible to adjust the amount and discharge rate of the medicament delivered to the target site of the subject, thereby preventing excessive supply of the medicament and expecting an appropriate therapeutic effect.

### BRIEF DESCRIPTION OF THE DRAWINGS

FIGS. 1 and 2 are flowcharts of a method for designing a mouth shield having a medicament application area according to one embodiment of the present invention.
FIG. 3 is a view showing an example in which a tooth part and a gum part are classified and identified in oral scan data according to one embodiment of the present invention.
FIGS. 4 to 6 are schematic views of the mouth shield having the medicament application area according to one embodiment of the present invention.
FIG. 7 is a view showing an example of mechanism in which a medicament applied to a medicament application surface is delivered to a target site according to one embodiment of the present invention.
FIG. 8 is a view showing an example of an internal configuration of a computing device according to one embodiment of the present invention.

### DETAILED DESCRIPTION OF THE INVENTION

Hereinafter, various embodiments and/or aspects will be disclosed with reference to drawings. In the following description, multiple concrete details will be disclosed in order to help general understanding of one or more aspects for the purpose of description. However, it will also be appreciated by those skilled in the art to which the present invention pertains that such aspect(s) may be practiced without the specific details. In the following description and accompanying drawings, specific exemplary aspects of one or more aspects will be described in detail. However, the aspects are exemplary, and some equivalents of various aspects may be used, and the descriptions herein are intended to include both the aspects and equivalents thereto.

It is not intended that any "embodiment", "example", "aspect", "illustration", and the like used in the specification is preferable or advantageous over any other "embodiment", "example", "aspect", "illustration", and the like.

Further, the terms "includes" and/or "including" mean that a corresponding feature/or component exists, but it should be appreciated that the terms "include" or "including" mean that presence or addition of one or more other features, components, and/or a group thereof is not excluded.

Further, terms including an ordinal number such as "first" or "second' may be used for the names of various components, not limiting the components. The above terms are used merely for the purpose of distinguishing one element from another element. For example, a first component may be referred to as a second component and vice versa without departing the scope of the present invention. The term "and/or" includes a combination of a plurality of related enumerated items or any of the plurality of related enumerated items.

In addition, unless otherwise defined, all terms used herein, including technical or scientific terms, have the same meaning as commonly understood by one of ordinary skill in the art to which this invention belongs. Terms as those defined in generally used dictionaries are to be interpreted to have the meanings consistent with the contextual meanings in the relevant field of art, and are not to be interpreted to have idealistic or excessively formalistic meanings unless explicitly defined in the embodiments of the present invention.

The present invention relates to a method for designing a mouth shield having a medicament application area, and more specifically, an object of the present invention is to achieve the purpose of enhancing oral health of a user who wears a mouth shield by continuously supplying a medicament (M) that is prepared to enhance oral health in the oral cavity to a target site set in the oral cavity.

Meanwhile, it should be understood that the present invention is a technology related to a structure and design method of the mouth shield having the medicament application area, and does not include clinical treatment and does not include medical treatment or clinical treatment and methods for humans.

Hereinafter, a detailed description of the present invention for achieving the above object will be described with reference to the accompanying drawings, and a plurality of drawings may be referenced simultaneously to describe one or more technical features or components constituting the present invention.

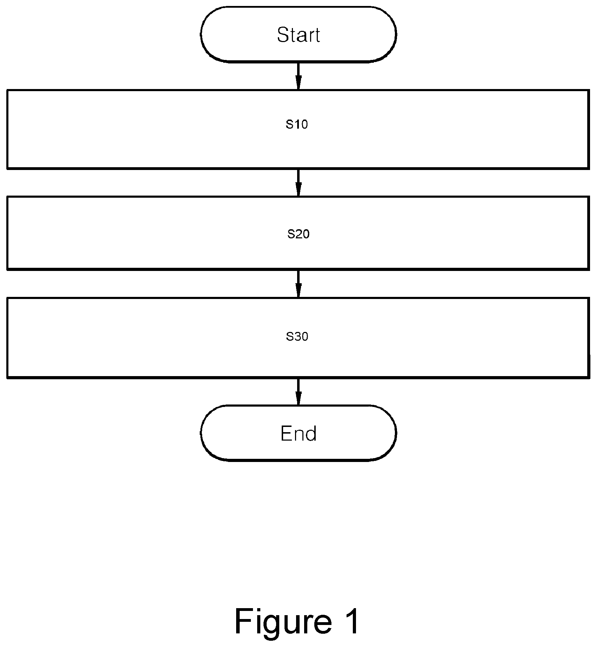

First, referring to FIG. 1, it can be seen that FIG. 1 is a flowchart showing a method for designing a mouth shield 100 having a medicament application area

As shown in FIG. 1, according to the present invention, an object classification and identification step (S10) of classifying and identifying objects corresponding to a tooth part (30T) and a gum part (30G) from oral scan data (1), which is obtained by three-dimensionally scanning an oral condition of a subject, using a preset object extraction algorithm, is performed.

In this case, the oral scan data (1) described in object classification and identification step (S10) may be understood as a concept of data obtained by scanning the oral cavity of the subject using a 3D oral cavity scanner (intraoral scanner, CBCT, etc.), and a file format thereof may be an STL file format and may follow a file format having the best compatibility in a 3D graphic software.

In addition, the oral scan data (1) includes information about teeth, gums, and oral structures, and according to the present invention, the object extraction algorithm is applied to the oral scan data (1) to identify the tooth part (30T) and the gum part (30G) of the subject.

As one embodiment, the object extraction algorithm used in the present invention undergoes a process of locating a circle at the center of a simple coordinate of an image of the oral scan data (1) and gradually increasing the size of the circle to search for a contact point for initial contact with the teeth or gums. Accordingly, the object extraction algorithm may scatter microspheres at regular intervals based on the initial contact point so that the microspheres may be located in each tooth, thereby identifying the location and outline of the teeth and securing basic data capable of searching for boundaries between the teeth and the gums.

Meanwhile, after searching for the initial contact point, a process of arranging one or more spheres (sizes of 1/10,000 mm) at regular intervals inside the teeth, detecting an event in which the spheres are randomly moved at a constant speed to escape from the inside of the teeth to the outside, and replicating the spheres at regular cycles to uniformly distribute the spheres inside the teeth, is performed.

However, in this case, an STL file-based three-dimensional model is formed of triangular meshes so that a mesh data direction is reversed, a relationship between the inside and the outside of the teeth is clearly set by reversing a direction vector of each triangular mesh, and the sizes of the replicated spheres are gradually increased in the process of generating the plurality of spheres in a distributed manner so that representative spheres are evenly arranged on individual teeth.

According to the performance of the process, spheres corresponding to 14 teeth of each of a lower jaw and an upper jaw are generated, and thereafter, when the coordinates represented by the spheres are derived, representative coordinates of each tooth are derived, and a contact point of the teeth is derived through the contact point of the spheres, so that the coordinates of the teeth and feature data about the contact point may be extracted.

In addition, according to the present invention, information about the position, rotation, and size of the teeth may be extracted based on the coordinates of the representative sphere, and axis data for each tooth may be generated so as to be parallel to a root direction of the teeth, thereby securing individual feature data of each tooth and completing the object classification between the tooth part (30T) and the gum part (30G) of the subject.

Meanwhile, according to the present invention, it is possible to derive an image of each individual tooth through a mesh separation program based on a SplitDisjointMesh function in a program such as an Rhino 3D work tool, and it is possible to generate data including each tooth data, feature data of teeth, and an axis of teeth by processing a tooth image by deriving the axis of teeth based on mesh information for each tooth for the tooth image, and it is possible to clearly identify an object corresponding to the tooth part (30T) by automatically deriving a volume center point and an area center point from the mesh information and setting the axis of the teeth.

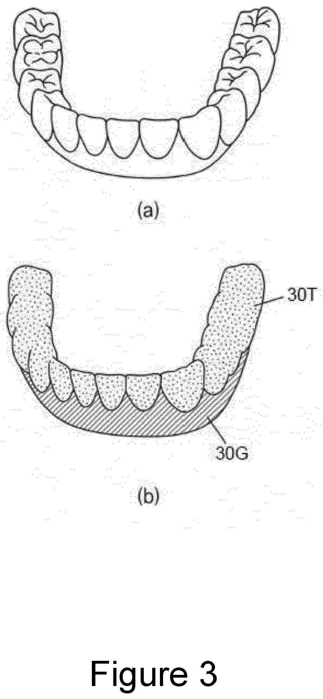

Meanwhile, FIG. 3(a) shows an example of the oral scan data (1) about the lower jaw among the oral scan data (1) of the subject, and FIG. 3(b) shows an example of the tooth part (30T) and the gum part (30G) for the lower jaw of the subject, which are classified and identified using the object extraction algorithm.

Obviously, according to another embodiment of the present invention, various known technologies such as a technology of dividing a boundary between the tooth part (30T) and the gum part (30G) using a machine learning-based 3D object extraction algorithm (CNN, PointNetemd) and an edge detection algorithm may be applied, but the present invention is not limited thereto.

After object classification and identification step (S10), a temporary model generation step (S20) of generating a temporary model by performing modeling using a design program on a mouth shield (100) having a shape to cover the tooth part (30T) and the gum part (30G) identified in object classification and identification step (S10), is performed.

In this case, as the above-described design program, a CAD software program including at least one of AutoCAD, Blender, and Rhino may be used, and non-uniform rational B-splines (NURBS)-based surface modeling is performed to perform modeling on the mouth shield (100) corresponding to contours of the tooth part (30T) and the gum part (30G) of the subject.

However, in this case, the mouth shield (100) may have a specific shape that surrounds the entire tooth part (30T) and the gum part (30G) may have a shape including a gingival marginal (or marginal gingival) line, and this may be understood as being because a medicament application surface (101) to be described later is set as an area corresponding to the gingival marginal line.

Meanwhile, when the temporary model is generated in temporary model generation step (S20), a correction model generation step (S30) of generating a correction model, in which correction is performed on the temporary model when the temporary model is generated in temporary model generation step (S20), a design model for the temporary model is corrected such that the medicament application surface (101) having a preset thickness is secured in consideration of the application thickness of the medicament (M) to be applied to an inner area of the mouth shield (100), thereby generating the correction model with optimized consistency, is performed.

Specifically, in correction model generation step (S30), the correction model may be generated by changing the design such that a predetermined separation space for securing the medicament application surface (101) is formed in a direction including at least one of a lingual side and a labial side of the tooth part (30T) in the inner area of the temporary model.

In this case, in correction model generation step (S30) described above, geometric design correction may be simply performed, but preferably, in addition to performing the geometric design correction, an AI-based modeling technology is applied to generate the correction model with optimized consistency. In more detail, by utilizing the oral scan data (1) and oral examination information of the subject (for example, age, body weight, gum condition, severity of disease, etc. of the subject) as learning data, a prediction model based on machine learning or deep learning may automatically calculate an optimal thickness, separation distance, and position range of the medicament application surface, and design correction for the temporary model is performed based on the data.

For example, when the gum condition of the subject is classified as "medium-term periodontitis" and a time required for delivering a medicament (M) is set as "long-term", the AI-based prediction model may automatically derive a correction proposal for increasing the thickness of the medicament application surface compared to the existing set thickness and reflect the correction proposal in the design of the correction model.

In addition, in correction model generation step (S30), a method for predicting the thickness of the medication application surface corresponding to data of the subject using a regression-based machine learning model or automatically detecting the gingival margin part and a lesion site using a CNN or PointNet type 3D deep learning network and determining the range and shape of a separation space according to the prediction or detection may be applied.

In addition, according to another embodiment of the present invention, an optimal design of the correction model may be induced by repeatedly learning design parameters based on application stability, wearing consistency, medicament absorption efficiency, and the like as a comprehensive index by using a reinforcement learning-based optimization algorithm (for example, Proximal Policy Optimization), and according to the present invention, the design customized for anatomical and physiological characteristics and the treatment purpose of each subject is possible by generating the AI-based correction model, thereby contributing to greatly improving medicament (M) delivery efficiency and therapeutic effect.

In another embodiment, the correction model in correction model generation step (S30) may be generated by performing a design change of adjusting an inner space of the mouth shield (100) such that the medicament (M) may be applied at a preset thickness (for example, 0.5 mm).

In addition, the medicament application surface (101) described in the present invention may refer to the entire inner area of the mouth shield (100) surrounding the tooth part (30T), and accordingly, the correction model performs a design change such that the thickness of the entire inner surface of the mouth shield (100) is spaced apart from the tooth part (30T) by a predetermined distance in the temporary model, and the medicament application surface (101) configured as described above is suitable for medicament application for tooth whitening, oral protection and hydration or a wide range of antimicrobial treatments.

As still another embodiment of the present invention, in order to supply the medicament (M) to an area corresponding to a specific target site of teeth tissue (for example, the gingival margin part), the medicament application surface (101) described in the present invention may provide a predetermined separation space corresponding to the gingival margin line and formed in a direction toward the lingual side of the teeth (i.e., the inside of the teeth), and may be determined as the medicament application surface (101) to generate the correction model.

As still another embodiment of the present invention, in order to supply the medicament (M) to an area corresponding to a specific target site of teeth tissue, the medicament application surface (101) described in the present invention may have a predetermined separation space as in the schematic view of FIG. 3 in a direction toward the labial side of the teeth (i.e., the outside of the teeth), and the predetermined separation space may be determined as the medicament application surface (101), thereby generating the correction model.

Obviously, as still another embodiment of the present invention, the medicament application surface (101) described in the present invention is not limited to an area corresponding to the gingival margin part in the inner area of the temporary model, but corresponding to any one of the directions toward the lingual and labial sides of the tooth part (30T), but may have predetermined separation spaces in both directions corresponding to the lingual and labial sides, and each of the predetermined separation spaces may be determined as the medicament application surface (101), thereby generating the correction model.

That is, the medicament application surface (101) is not selectively formed in an area corresponding to either the lingual side or the labial side, but is formed in areas corresponding to both the lingual side and the labial side such that the medicament (M) is uniformly supplied to the entire periodontal tissue surrounding the teeth, thereby maximizing effects of alleviating inflammation and removing bacteria, and particularly, expecting to obtain excellent effects in reducing inflammation and pain occurring during orthodontic treatment.

Meanwhile, when the medicament application surface (101) described above is formed in a limited form in the specific target site of the teeth tissue to supply the medicament (M) to the subject in a local range, it is possible to intensively treat diseases (for example, periodontitis or gingivitis) occurring in the gingival margin part, and it is possible to prevent the medicament (M) from being spread into unnecessary sites in the tooth part (30T) or oral mucosa.

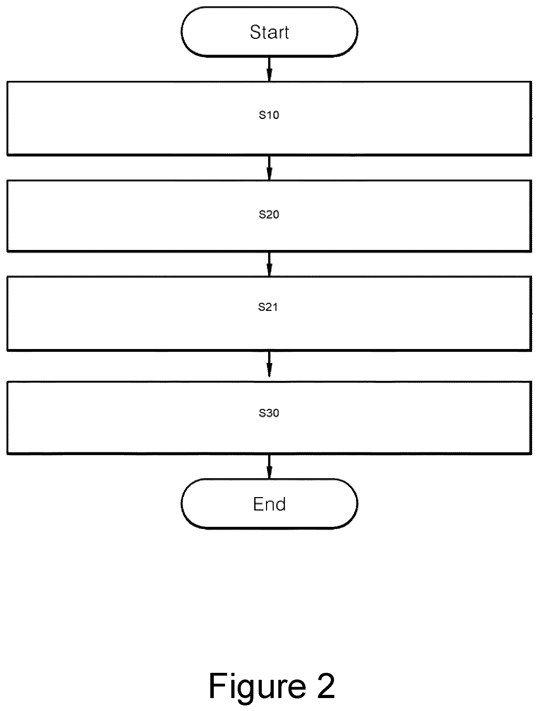

However, in the forming of the medicament application surface (101) in correction model generation step (S30) of the present invention, the present invention may further include an oral examination information collection step (S21) of collecting oral examination information including at least one of age, body weight, gum condition, and severity of a disease of the subject as the oral examination information of the subject, before the correction model generation step step (S30) shown in FIG. 2.

In oral examination information collection step (S21) described above, it may be understood that the reason for considering the age of the subject described above is that the tissue condition of the gums and the periodontal varies depending on children, adolescents, adults, and the elderly, and it may be understood that the reason for considering the body weight is that the absorption and metabolism rate of the medicament (M) may vary depending on the body weight.

In addition, considering the gum condition may be understood as being because a medicament (M) of a specific component is required when the subject has gum diseases such as gingivitis and periodontitis, and it may be understood as a configuration for providing a personalized medicament (M) supply unit to the subject by considering that the concentration of the medicament (M) may vary depending on the initial, intermediate, and terminal stages.

That is, according to the present invention, oral examination information collection step (S21) is performed to calculate what kind of medicament (M) is required for the subject and what is the appropriate dose of the medicament (M), and accordingly, in correction model generation step (S30), the thickness of the medicament application surface (101) corresponding to the calculated dose of the medicament (M) is determined based on the performance result in oral examination information collection step (S21) to correct the design of the temporary model.

As one embodiment of analyzing the required amount of medicament (M) according to age, assuming that there are a subject A who is 10 years old and a subject B who is 50 years old, according to the present invention, since the periodontal tissue is weak in the process of growing baby teeth or permanent teeth, the thickness of the medicament application surface (101) may also be set to be a value (0.3 mm) that is relatively smaller than a set value (0.5 mm) to prevent unnecessary absorption of the medicament (M), and since the subject B has thicker gums and more mature periodontal tissue, the medicament (M) concentration may be set to be relatively high and the thickness of the medicament application surface 101 may be set to be a value (0.7 mm) that is relatively larger than the set value to maximize the continuous medicament (M) discharge effect.

In addition, as one embodiment of analyzing a required amount of a medicament (M) according to body weight, assuming that there are a subject A having a body weight of 45 Kg and a subject B having a body weight of 90 Kg, according to the present invention, in the case of the subject A predicted to have a fast medicament absorption rate due to a relatively low metabolic rate, since there is a possibility of a medicament (M) side effect due to an unnecessary increase in absorption when an excessive amount of the medicament (M) is applied, the thickness of the medicament (M) applied to the medicament application surface (101) may be adjusted to 0.4 mm, which is a thickness relatively smaller than the set value (0.5 mm), and in the case of the subject B predicted to have a relatively high metabolic rate and the medicament (M) to be relatively rapidly decomposed, the medicament application surface (101) may be designed such that the application thickness of the medicament (M) is 0.6 mm, which is relatively larger than the set value (0.5 mm), in order to increase a discharge duration time of the medicament (M).

In addition, as another embodiment of the design of the medicament application surface (101) in consideration of the gum condition, assuming that there are a subject A who has healthy gums, a subject B who has early stage periodontitis, and a subject C who has intermediate stage periodontitis, since the medicament (M) is supplied to the subject A for the preventive purpose, the thickness of the medicament application surface (101) is designed to be 0.3 mm, which is a thickness smaller than the set value (0.5 mm), the subject B needs treatment for periodontitis, but since the condition is mild, the medicament application surface (101) may be designed to be 0.5 mm, which is a thickness corresponding to the set value, and since the subject C is in an urgent and relatively serious condition, the thickness of the medicament application surface (101) is adjusted to 0.8 mm, so that the medicament (M) may be supplied to the gingival margin part for a long time, thereby maximizing the therapeutic effect.

Meanwhile, according to correction model generation step (S30), a 3D printing-based molding process is performed based on the design data of the correction model generated in correction model generation step (S30), thereby manufacturing a real mouth shield (100).

In this case, it is preferable that the real mouth shield (100) is formed in a 3D printing laboratory, which is a specialized production facility for providing a customized medical device using a 3D printer (21), so that the mouth shield (100) having high precision with the design data is manufactured, and as shown in FIG. 3, the generated real mouth shield (100) has a medicament application surface (101) formed on the mouth shield (100) and applies a medicament (M) to the corresponding area to deliver the medicament (M) to the subject.

In addition, as another preferred embodiment of the present invention, it is preferable that the real mouth shield (100) described in the present invention is formed of a urethane-based material having excellent biocompatibility. Specifically, according to the present invention, when the real mouth shield (100) is manufactured, soft urethane (for example, urethane having a hardness of about 70 to 90 based on Shore A hardness) is used in an area directly in contact with an outer surface of the tooth part (30T) to surround the tooth part (30T), and as a material forming the outer surface of the mouth shield (100) while surrounding the soft urethane, hard urethane (for example, urethane having a hardness of about 30 to 80 based on Shore D hardness) is used, so that urethane materials having mutually different physical properties have a multi-layer structure and are physically or chemically combined to provide comfortable wearing sensation and increased durability when worn in the oral cavity of the subject, but the present invention is not limited thereto.

Meanwhile, after the real mouth shield (100) is manufactured as described above, the medicament (M) may be applied to the medicament application surface (101) of the mouth shield (100) to be simply provided to the subject, but preferably, the medicament application surface (101) formed on the mouth shield (100) includes a release film disposed in the form in which the medicament (M) applied to the medicament application surface (101) is tightly covered.

In this case, the above-described release film has a structure in which the inside thereof includes a plurality of pores, and serves to deliver the medicament (M) applied to the medicament application surface (101) to the target site including the tooth part (30T) and the gum part (30G) of the subject through the plurality of pores (micro pores having a size of 100 to 300 µm), and the release film provides an additional function of preventing the medicament (M) applied to the medicament application surface (101) from being rapidly washed down or diluted by saliva present in the oral cavity of the subject.

To this end, the release film described in the present invention may be adhered to an inner surface of the mouth shield (100) formed of urethane with unique viscosity by applying a bioadhesive polymer (hyaluronic acid, carboxymethyl cellulose) to an edge line covering the medicament application surface (101).

Obviously, according to the present invention, a medical silicone adhesive layer is formed on the edge line covering the medicament application surface (101), so that the release film may have adhesiveness with the inner surface of the mouth shield (100) and minimize irritation to other skin tissues or mucous membranes, but the present invention is not limited thereto.

In addition, according to another preferred embodiment of the present invention, in forming the above-described release film, the release film may be configured in a multi-layer structure rather than a single layer structure to further increase medicament (M) delivery efficiency.

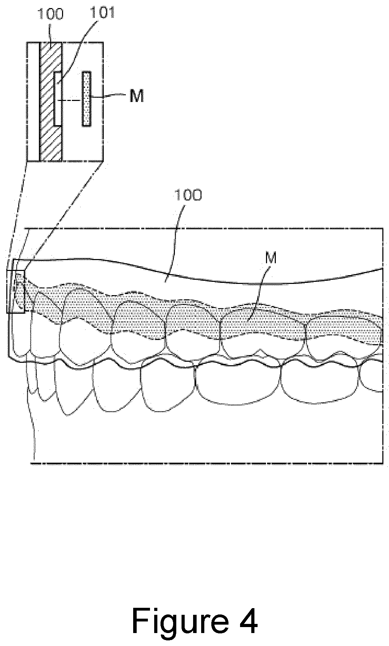

Simultaneously referring to FIG. 4, the above-described release film may be configured to have a multi-layer structure including an outer layer (L1) formed of a material including at least one of a polyurethane membrane and a fluororesin and having a structure including a plurality of pores, and a hydrophilic hydrogel-based absorption layer (L2) serving to adjust a discharge rate of the medicament (M) by absorbing and storing the medicament (M) applied to the medicament application surface (101) between the medicament application surface (101) and the outer layer (L1).

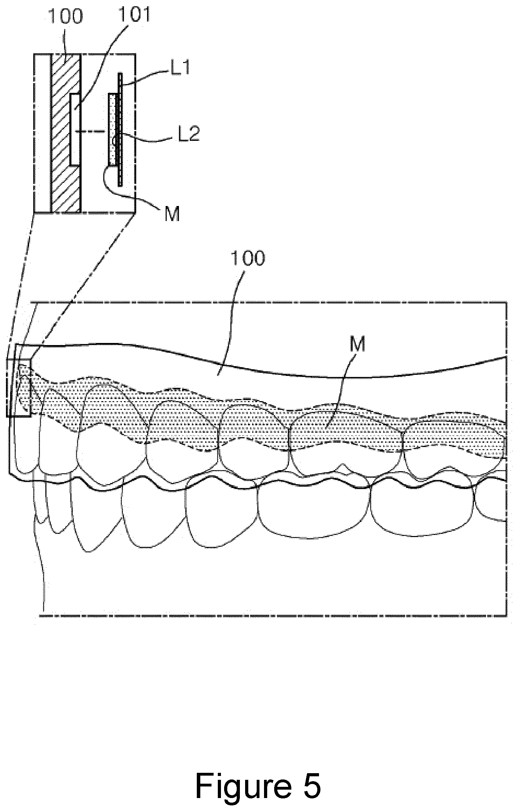

In this case, the above-described absorbent layer (L2) serves to store the medicament (M) and gradually discharge the medicament (M) through the pore structure of the outer layer (L1) as shown in FIG. 5, so that a continuous medicament (M) delivery effect may be expected for a long time, and compared to the single layer structure, the medicament (M) is gradually discharged whenever necessary while maintaining the antibacterial effect for a long time, so that improvement of the therapeutic effect may be expected by delivering the medicament (M) at a constant concentration.

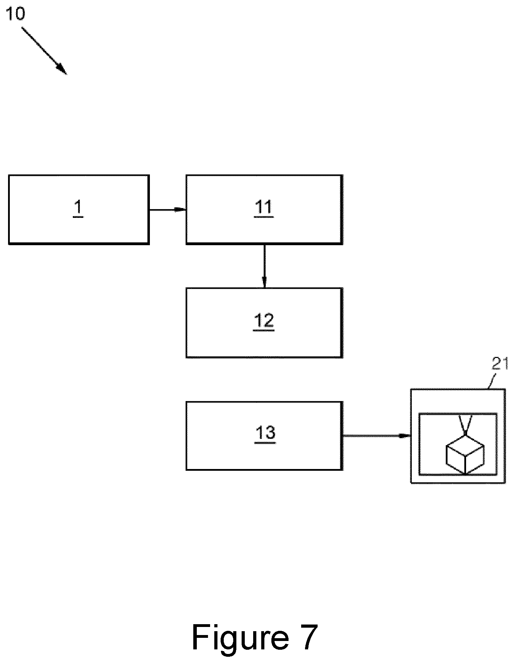

Meanwhile, referring to 10 of FIG. 7, it can be seen that FIG. 7 shows a configuration view of an apparatus (10) for designing the mouth shield (100) having the medicament application area according to one embodiment of the present invention.

As shown in FIG. 7, the apparatus (10) of the present invention may include an object classification and identification unit (11), a temporary model generation unit (12), and a correction model generation unit (13) as main components.

Specifically, the object classification and identification unit (11) serves to classify and identify objects corresponding to a tooth part (30T) and a gum part (30G) from oral scan data (1), which is obtained by three-dimensionally scanning an oral condition of a subject, using a preset object extraction algorithm.

That is, the object classification and identification unit (11) may be understood as being capable of performing all the functions performed in object classification and identification step (S10) of FIG. 1 described above, and according to the present invention, an oral structure of the subject may be clearly recognized by performing the function of the object classification and identification unit (11), and thus the mouth shield (100) having high consistency may be designed.

The temporary model generation unit (12) serves to generate a temporary model by performing modeling using a design program on the mouth shield (100) having a shape to cover the tooth part (30T) and the gum part (30G) identified by the object classification and identification unit (11).

That is, the temporary model generation unit (12) may be understood as being capable of performing all the functions performed in temporary model generation step (S20) of FIG. 1, and according to the present invention, by performing the function of the temporary model generation unit (12), modeling of the mouth shield (100) including the medicament application area may be performed.

In addition, the correction model generation unit (13) serves to generate a correction model, in which correction is performed on the temporary model generated by the temporary model generation unit (12), and a design for the temporary model is corrected such that the medicament application surface (101) having a preset thickness is secured in consideration of the application thickness of the medicament (M) to be applied to an inner area of the mouth shield (100), thereby generating the correction model with optimized consistency.

That is, the correction model generation unit (13) may be understood as being capable of performing all the functions performed in correction model generation step (S30) of FIG. 1, and according to the present invention, by performing the function of the correction model generation unit (13), it is possible to manufacture the mouth shield (100) having excellent consistency with the oral structure of the subject, a medicament application surface, and a medicament (M) delivery unit.

Meanwhile, although not explicitly shown in FIG. 7, in another embodiment of the apparatus (10) of the present invention, an oral examination information collection unit may be further included together with the object classification and identification unit (11), the temporary model generation unit (12), and the correction model generation unit (13) described above.

In this case, the oral examination information collection unit serves to collect oral examination information including at least one of age, body weight, gum condition, and severity of a disease of the subject as the oral examination information of the subject, before performing the function of the correction model generation unit (13), so that the oral examination information collection unit performs a function of generating a correction model that is more optimized for the oral examination condition of the subject when the correction model generation unit (13) generates the correction model by processing the temporary model.

As a result, it may be understood that the oral examination information collection unit may perform all the functions performed in oral examination information collection step (S21) of FIG. 2, and unnecessary descriptions of the overlapping embodiments described above will be omitted.

According to one embodiment of the present invention described comprehensively above, in order to enhance oral health in the oral cavity, the medicament (M) applied to the medicament application surface 101 of the mouth shield (100) is continuously delivered to the target site of the subject, so that a therapeutic effect may be maintained for a long time compared to a method for supplying a medicament (M) like gargling, thereby rapidly achieving the purpose of enhancing oral health.

In addition, according to one embodiment of the present invention, it is possible to expect an efficient local therapeutic effect by setting the medicament application surface (101) formed on the mouth shield (100) as a surface corresponding to the gingival margin line of the subject.

In addition, according to one embodiment of the present invention, the present invention does not simply apply the medicament (M) to the medicament application surface (101) formed on the mouth shield (100) to provide the medicament (M) to the subject, but applies the medicament (M) to the medicament application surface (101) and tightly covers the medicament application surface (101) with a release film, so that it is possible to adjust the amount and discharge rate of the medicament (M) delivered to the target site of the subject, thereby preventing excessive supply of the medicament (M) and expecting an appropriate therapeutic effect.

While the embodiments have been described with reference to limited examples and drawings as described above, it will be apparent to one of ordinary skill in the art that various changes and modifications may be made from the above description.

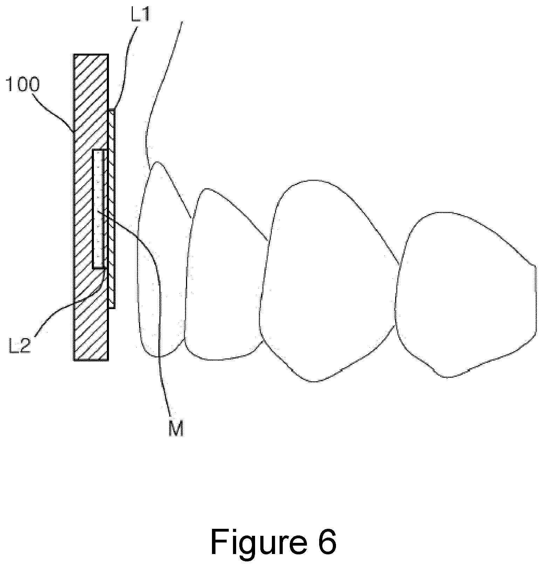

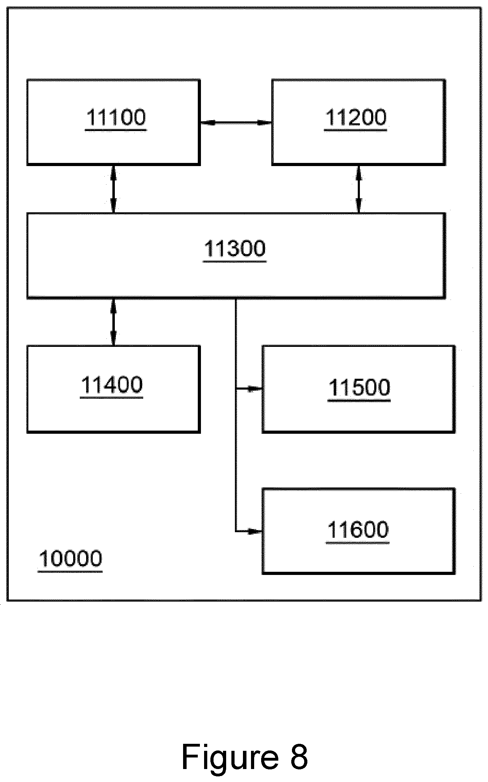

Meanwhile, referring to FIG. 8, FIG. 8 is a view showing an example of an internal configuration of the computing device according to one embodiment of the present invention. In the following description, redundant descriptions of the embodiment corresponding to the above descriptions for FIGS. 1 to 7 will be omitted.

As shown in FIG. 8, a computing device (10000) may at least include at least one processor (11100), a memory (11200), a peripheral interface (11300), an input/output (I/O) subsystem (11400), a power circuit (11500), and a communication circuit (11600). In this case, the computing device (10000) may correspond to a user terminal A connected to a tactile interface device or correspond to the above-described computing device B.

The memory (11200) may include, for example, a high-speed random access memory, a magnetic disk, an SRAM, a DRAM, a ROM, a flash memory, or a non-volatile memory. The memory (11200) may include a software module, an instruction set, or other various data necessary for the operation of the computing device (10000).

In this case, access to the memory (11200) from other components of the processor (11100) or the peripheral interface (11300), may be controlled by the processor (11100).

The peripheral interface (11300) may combine an input and/or output peripheral device of the computing device (10000) to the processor (11100) and the memory (11200). The processor (11100) may execute the software module or the instruction set stored in the memory (11200), thereby performing various functions for the computing device (10000) and processing data.

The input/output subsystem (11400) may combine various input/output peripheral devices to the peripheral interface (11300). For example, the input/output subsystem (11400) may include a controller for combining the peripheral device such as monitor, keyboard, mouse, printer, or a touch screen or sensor, if needed, to the peripheral interface (11300). According to another aspect, the input/output peripheral devices may be combined to the peripheral interface (11300) without passing through the input/output subsystem (11400).

The power circuit (11500) may provide power to all or a portion of the components of the terminal. For example, the power circuit (11500) may include a power failure detection circuit, a power converter or inverter, a power status indicator, a power failure detection circuit, a power converter or inverter, a power status indicator, or arbitrary other components for generating, managing, or distributing power.

The communication circuit (11600) may use at least one external port to enable communication with other computing devices.

Alternatively, as described above, the communication circuit (11600) may include an RF circuit, if needed, to transmit and receive an RF signal, also known as an electromagnetic signal, thereby enabling communication with other computing devices.

The above embodiment of FIG. 8 is merely an example of the computing device (10000), and the computing device (11000) may have a configuration or arrangement in which some components shown in FIG. 8 are omitted, additional components not shown in FIG. 8 are further provided, or at least two components are combined. For example, a computing device for a communication terminal in a mobile environment may further include a touch screen, a sensor, or the like, in addition to the components shown in FIG. 8. The communication circuit (11600) may include a circuit for RF communication of various communication schemes (such as WiFi, 3G, LTE, Bluetooth, NFC, and Zigbee). The components that may be included in the computing device (10000) may be implemented by hardware, software, or a combination of both hardware and software which include at least one integrated circuit specialized in a signal processing or an application.

The methods according to the embodiments of the present invention may be implemented in the form of program instructions to be executed through various computing devices so as to be recorded in a computer-readable medium. In particular, a program according to the embodiment of the present invention may be configured as a PC-based program or an application dedicated to a mobile terminal. The application to which the present invention is applied may be installed in a user terminal through a file provided by a file distribution system. For example, a file distribution system may include a file transmission unit (not shown) that transmits the file according to the request of the user terminal.

The above-described device may be implemented by hardware components, software components, and/or a combination of hardware components and software components. For example, the devices and components described in the embodiments may be implemented by using at least one general purpose computer or special purpose computer such as a processor, a controller, an arithmetic logic unit (ALU), a digital signal processor, a microcomputer, a field programmable gate array (FPGA), a programmable logic unit (PLU), a microprocessor, or any other device capable of executing and responding to instructions. The processing device may execute an operating system (OS) and one or more software applications executed on the operating system.

In addition, the processing device may access, store, manipulate, process, and create data in response to the execution of the software. For the further understanding, in some cases, one processing device may be used, however, those skilled in the art will be appreciated that the processing device may include a plurality of processing elements and/or a plurality of types of processing elements. For example, the processing device may include a plurality of processors or one processor and one controller. In addition, other processing configurations, such as a parallel processor, are also possible.

The software may include a computer program, a code, an instruction, or a combination of at least one thereof, may configure the processing device to operate as desired, or may instruct the processing device independently or collectively. In order to be interpreted by the processor or to provide instructions or data to the processor, the software and/or data may be permanently or temporarily embodied in any type of machine, component, physical device, virtual equipment, and computer storage medium or device. The software may be distributed over computing devices connected to networks, so as to be stored or executed in a distributed manner. The software and data may be stored in at least one computer-readable recording medium.

The above-described embodiments of the present invention may be recorded in computer-readable media including program instructions to implement various operations embodied by a computer. The computer-readable medium may also include, alone or in combination with the program instructions, data files, data structures, and the like. The program instructions recorded on the media may be those specially designed and constructed for the purposes of the embodiments, or they may be of the kind well-known and available to those having skill in the computer software arts. Examples of computer-readable recording media include magnetic media such as hard disks, floppy disks, and magnetic tape; optical media such as CD ROM disks and DVDs; magneto-optical media such as optical discs; and hardware devices that are specially configured to store and perform program instructions, such as read-only memory (ROM), random access memory (RAM), flash memory, and the like.

Examples of program instructions include both machine code, such as produced by a compiler, and files containing higher level code that may be executed by the computer using an interpreter. The described hardware devices may be configured to act as one or more software modules in order to perform the operations of the above-described embodiments of the present invention, or vice versa.

While the embodiments have been described with reference to limited examples and drawings as described above, it will be apparent to one of ordinary skill in the art that various changes and modifications may be made from the above description. Suitable results may be achieved if the described techniques are performed in a different order, and/or if components in a described system, architecture, device, or circuit are combined in a different manner, and/or replaced or supplemented by other components or their equivalents. Therefore, other implementations, other embodiments, and equivalents of the claims are within the scope of the following claims.

### REFERENCE NUMBERS

1 Oral scan data
10 Apparatus
11 Object classification and identification unit
12 Temporary model generation unit
13 Correction model generation unit 21 3D printer
100 Mouth shield
101 Medicament application surface
10000 Computing device
11100 Processor
11200 Memory
11300 Peripheral interface
11400 Input/output (I/O) subsystem
11500 Power circuit
11600 Communication circuit
L1 Outer layer
L2 Absorbent layer
M Medicament
30T Tooth part
30G Gum part
S10 Object classification and identification step
S20 Temporary model generation step
S21 Oral examination information collection step
S30 Correction model generation step

## Claims

1. A method for designing a mouth shield having a medicament application area, which is implemented by a computing device (10000) including one or more processors (11100)and one or more memories (11200)for storing instructions executable in the processors (11100), the method comprising:
an object classification and identification step (S10) of classifying and identifying objects corresponding to a tooth part (30T) and a gum part (30G) from oral scan data, which is obtained by three-dimensionally scanning an oral condition of a subject, using a preset object extraction algorithm;
a temporary model generation step (S20) of generating a temporary model by performing modeling using a design program on the mouth shield having a shape to cover the tooth part (30T) and the gum part (30G) identified in the object classification and identification step (S10); and
a correction model generation step (S30) of generating a correction model, in which correction is performed on the temporary model wherein an artificial intelligence model calculates an application thickness of a medicament (M) to be applied to an inner area of the mouth shield based on the oral scan data and oral examination information of the subject, and a design of the temporary model is corrected such that a medicament application surface having a thickness corresponding to the calculated result is secured, thereby generating the correction model with optimized consistency of the mouth shield (100), so that a 3D printing-based molding process is performed based on design data of the correction model,
wherein the medicament application surface formed on the mouth shield (100) includes a release film disposed in a form of tightly covering the medicament application surface,
wherein the release film has a structure in which an inside thereof includes a plurality of pores so that the medicament (M) applied to the medicament application surface is delivered to the tooth part (30T) and the gum part (30G) of the subject through the plurality of pores, and
wherein the release film includes:
an outer layer (L1) formed of a material including at least one of a polyurethane membrane and a fluororesin and having a structure including a plurality of pores; and
a hydrophilic hydrogel-based absorbent layer (L2) configured to adjust a discharge rate of the medicament (M) by absorbing and storing the medicament applied to the medicament application surface between the medicament application surface and the outer layer (L1).

2. The method of claim 1, further comprising an oral examination information collection step (S21) of collecting oral examination information including at least one of age, body weight, gum condition, and severity of a disease of the subject as the oral examination information of the subject, before the correction model generation step (S30),
so that an appropriate dose of a medicament (M) required for the subject is calculated according to the collected oral examination information, and in the correction model generation step (S30), the design of the temporary model is corrected by determining a thickness of the medicament application surface corresponding to the calculated dose of the medicament (M).

3. The method of claim 2, wherein in the correction model generation step (S30), the correction model is generated by changing the design such that a predetermined separation space for securing the medicament application surface is formed in a direction including at least one of a lingual side and a labial side of the tooth part (30T) in the inner area of the temporary model.

4. The method of claim 1, wherein the medicament application surface formed on the mouth shield is an area including at least one of an entire inner area forming a contact surface with the tooth part (30T) and the gum part (30G) of the subject or a local area corresponding to a gingival margin part, which is a boundary between the tooth part (30T) and the gum part (30G) of the subject.

5. An apparatus (10) for designing a mouth shield having a medicament application area, which is implemented by a computing device (10000) including one or more processors (11100) and one or more memories (11200) for storing instructions executable in the processors (11100), the apparatus (10) comprising:
an object classification and identification unit (11) configured to classify and identify objects corresponding to a tooth part (30T) and a gum part (30G) from oral scan data, which is obtained by three-dimensionally scanning an oral condition of a subject, using a preset object extraction algorithm;
a temporary model generation unit (12) configured to generate a temporary model by performing modeling using a design program on the mouth shield having a shape to cover the tooth part (30T) and the gum part (30G) identified by the object classification and identification unit (11); and
a correction model generation unit (13) configured to generate a correction model, in which correction is performed on the temporary model wherein an artificial intelligence model calculates an application thickness of a medicament (M) to be applied to an inner area of the mouth shield (100) based on the oral scan data (1) and oral examination information of the subject, and a design of the temporary model is corrected such that a medicament application surface having a thickness corresponding to the calculated result is secured, thereby generating the correction model with optimized consistency of the mouth shield (100), so that a 3D printing-based molding process is performed based on design data of the correction model,
wherein the medicament application surface formed on the mouth shield includes a release film disposed in a form of tightly covering the medicament application surface,
wherein the release film has a structure in which an inside thereof includes a plurality of pores so that the medicament (M) applied to the medicament application surface is delivered to the tooth part (30T) and the gum part (30G) of the subject through the plurality of pores, and
wherein the release film includes:
an outer layer formed of a material including at least one of a polyurethane membrane and a fluororesin and having a structure including a plurality of pores; and
a hydrophilic hydrogel-based absorbent layer configured to adjust a discharge rate of the medicament (M) by absorbing and storing the medicament (M) applied to the medicament application surface between the medicament application surface and the outer layer.

6. A computer-readable recording medium that stores instructions for allowing a computing device (10000) to perform the following steps, wherein the steps comprise:
an object classification and identification step (S10) of classifying and identifying objects corresponding to a tooth part (30T) and a gum part (30G) from oral scan data (1), which is obtained by three-dimensionally scanning an oral condition of a subject, using a preset object extraction algorithm;
a temporary model generation step (S20) of generating a temporary model by performing modeling using a design program on the mouth shield (100) having a shape to cover the tooth part (30T) and the gum part (30G) identified in the object classification and identification step (S10); and
a correction model generation step (S30) of generating a correction model, in which correction is performed on the temporary model wherein an artificial intelligence model calculates an application thickness of a medicament (M) to be applied to an inner area of the mouth shield (100) based on the oral scan data (1) and oral examination information of the subject, and a design of the temporary model is corrected such that a medicament application surface having a thickness corresponding to the calculated result is secured, thereby generating the correction model with optimized consistency of the mouth shield (100), so that a 3D printing-based molding process is performed based on design data of the correction model,
wherein the medicament application surface formed on the mouth shield (100) includes a release film disposed in a form of tightly covering the medicament application surface,
wherein the release film has a structure in which an inside thereof includes a plurality of pores so that the medicament (M) applied to the medicament application surface is delivered to the tooth part (30T) and the gum part (30G) of the subject through the plurality of pores, and
wherein the release film includes:
an outer layer formed of a material including at least one of a polyurethane membrane and a fluororesin and having a structure including a plurality of pores; and
a hydrophilic hydrogel-based absorbent layer configured to adjust a discharge rate of the medicament (M) by absorbing and storing the medicament (M) applied to the medicament application surface between the medicament application surface and the outer layer (L1).
